(19) **Europäisches Patentamt / European Patent Office / Office européen des brevets**

(11) **EP 4 785 945 A1**

(12) **EUROPEAN PATENT APPLICATION**
published in accordance with Art. 153(4) EPC

(43) Date of publication:
05.08.2026 Bulletin 2026/32

(21) Application number: 24872386.8

(22) Date of filing: 26.09.2024

(51) International Patent Classification (IPC):
**A61K 31/519** (2006.01)    **A61P 19/02** (2006.01)
**A61P 29/00** (2006.01)

(52) Cooperative Patent Classification (CPC):
**A61K 31/519; A61P 19/02; A61P 29/00**

(86) International application number:
**PCT/JP2024/034410**

(87) International publication number:
**WO 2025/070601 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 27.09.2023 JP 2023164203

(71) Applicant: Teijin Pharma Limited
Tokyo 100-0013 (JP)

(72) Inventors:
• TASAKI, Daisuke
Tokyo 100-0013 (JP)

• TAKESHITA, Chie
Tokyo 100-0013 (JP)
• ITO, Katsuaki
Tokyo 100-0013 (JP)
• ASANO, Satoshi
Tokyo 100-0013 (JP)
• TSUJIMOTO, Shunsuke
Tokyo 100-0013 (JP)

(74) Representative: Callaghan, Dayle Anne
Kilburn & Strode LLP
Lacon London
84 Theobalds Road
London WC1X 8NL (GB)

(54) **THERAPEUTIC AGENT FOR RHEUMATOID ARTHRITIS**

(57)    Provided is a highly safe therapeutic agent for rheumatoid arthritis that produces effects after a short administration, exhibits strong efficacy, and is effective for human rheumatoid arthritis. The therapeutic agent for rheumatoid arthritis contains (R)-1-(6-((6-(1-hydroxyethyl)-8-(isopropylamino)pyrido[3,4-d]pyrimidin-2-yl)amino)pyridin-3-yl)piperazin-2-one or a pharmaceutically acceptable salt thereof.

Figure 1

EP 4 785 945 A1

**Description**

**TECHNICAL FIELD**

The present invention relates to a therapeutic agent for rheumatoid arthritis containing, as an active ingredient, (R)-1-(6-((6-(1-hydroxyethyl)-8-(isopropylamino)  pyrido[3,4-d]pyrimidin-2-yl)amino)pyridin-3-yl)piperazin-2-one  or  a pharmaceutically acceptable salt thereof.

**BACKGROUND ART**

Rheumatoid arthritis is a chronic inflammatory systemic autoimmune disease of unknown etiology. Rheumatoid arthritis is characterized by symmetrical synovitis that leads to cartilage damage and joint destruction, and can be accompanied by a number of extra-articular symptoms. Rheumatoid arthritis is generally a progressive disease, and long-term sufferers are known to experience worsening symptoms, including arthritis, joint destruction, physical dysfunction, and reduced life expectancy. The disease can develop at any age, and the incidence rate is highest in people in their 40s to 60s. Anti-rheumatic drugs, also known as disease-modifying anti-rheumatic drugs (DMARDs), do not suppress the inflammation of rheumatoid arthritis itself, but control the activity of rheumatoid arthritis by modifying the immune abnormalities of rheumatoid arthritis. DMARDs are the first-line treatment for rheumatoid arthritis patients. DMARDs include methotrexate and salazosulfapyridine, which are administered to patients already diagnosed with rheumatoid arthritis, and biological preparations such as TNF inhibitors and IL-6 inhibitors, which are administered to patients who do not respond well to such drugs as methotrexate and salazosulfapyridine. However, the problem has arisen that, even with the use of these DMARDs, less than 40% of patients achieve remission of clinical symptoms, and it is not possible to completely prevent the progression of joint destruction. In addition, it is also considered important to provide rheumatoid arthritis patients with treatment that improves subjective symptoms such as pain, and there is a demand for drugs that reduce disease activity and pain from the early stages of administration of investigational drugs.

It is also known that Janus kinase (JAK) inhibitors such as filgotinib are effective for rheumatoid arthritis (Non-Patent Literature 1). However, there are problems with JAK inhibitors in that they require long-term administration to produce efficacy, and in that their efficacy cannot be considered sufficient.

Patent Literature 1 discloses a number of compounds that have CDK4/6-inhibiting activity, and states that they are useful as prophylactic or therapeutic agents for rheumatoid arthritis, arteriosclerosis, pulmonary fibrosis, cerebral infarction, or cancer. However, it does not specifically describe their therapeutic effects on rheumatoid arthritis in humans.

**LIST OF CITATIONS**

**Non-Patent Literature**

[Non-Patent Literature 1] Ann Rheum Dis 2017; 76: 1009-1019

**Patent Literature**

[Patent Literature 1] WO 2016/194831 A

**SUMMARY OF INVENTION**

**Problem to Be Solved by the Invention**

The problem to be addressed by the present invention is to provide a highly safe therapeutic agent for rheumatoid arthritis that produces effects after a short administration, exhibits strong efficacy, and is effective for human rheumatoid arthritis.

**Means to Solve the Problem**

As a result of diligent study, the present inventors found that a composition containing (R)-1-(6-((6-(1-hydroxyethyl)-8-(isopropylamino)pyrido[3,4-d]pyrimidin-2-yl)amino)pyridin-3-yl)piperazin-2-one (hereinafter also referred to as "Compound 1") in a predetermined amount has excellent clinical efficacy against rheumatoid arthritis in humans, produces effects after a short administration, and is safe, thereby arriving at the present invention.

Specifically, aspects of the present invention include the following.

[Aspect 1-1] A therapeutic agent for rheumatoid arthritis comprising (R)-1-(6-((6-(1-hydroxyethyl)-8-(isopropylamino) pyrido[3,4-d]pyrimidin-2-yl)amino)pyridin-3-yl)piperazin-2-one or a pharmaceutically acceptable salt thereof.

[Aspect 1-2] Use of (R)-1-(6-((6-(1-hydroxyethyl)-8-(isopropylamino)pyrido[3,4-d]pyrimidin-2-yl)amino)pyridin-3-yl) piperazin-2-one or a pharmaceutically acceptable salt thereof in the manufacture of a therapeutic agent for rheumatoid arthritis.

[Aspect 1-3] A method for treating rheumatoid arthritis comprising administering, to a subject suffering from rheumatoid arthritis, (R)-1-(6-((6-(1-hydroxyethyl)-8-(isopropylamino)pyrido[3,4-d]pyrimidin-2-yl)amino)pyridin-3-yl)piperazin-2-one or a pharmaceutically acceptable salt thereof.

[Aspect 1-4] The compound (R)-1-(6-((6-(1-hydroxyethyl)-8-(isopropylamino)pyrido[3,4-d]pyrimidin-2-yl)amino)pyridin-3-yl)piperazin-2-one or a pharmaceutically acceptable salt thereof for use in treating rheumatoid arthritis.

[Aspect 2] The therapeutic agent for rheumatoid arthritis according to Aspect 1-1, the use according to Aspect 1-2, the method according to Aspect 1-3, or the compound according to Aspect 1-4, wherein the daily dosage of the compound is from 10 to 200 mg.

[Aspect 3] The therapeutic agent for rheumatoid arthritis according to Aspect 1-1, the use according to Aspect 1-2, the method according to Aspect 1-3, or the compound according to Aspect 1-4, wherein the daily dosage of the compound is from 30 to 125 mg.

[Aspect 4] The therapeutic agent for rheumatoid arthritis according to any one of Aspect 1-1 through Aspect 3, the use according to any one of Aspect 1-2 through Aspect 3, the method according to any one of Aspect 1-3 through Aspect 3, or the compound according to Aspect 1-4 through Aspect 3, which is to be administered once a day.

[Aspect 5] The therapeutic agent for rheumatoid arthritis according to any one of Aspect 1-1 through Aspect 4, the use according to any one of Aspect 1-2 through Aspect 4, the method according to any one of Aspect 1-3 through Aspect 4, or the compound according to any one of Aspect 1-4 through Aspect 4, which is to be used in combination with an additional therapeutic agent for rheumatoid arthritis.

[Aspect 6] The therapeutic agent for rheumatoid arthritis, the use, the method, or the compound according to Aspect 5, wherein the "additional therapeutic agent for rheumatoid arthritis" is a disease-modifying anti rheumatic drug (DMARD).

[Aspect 7] The therapeutic agent for rheumatoid arthritis, the use, the method, or the compound according to Aspect 5, wherein the "additional therapeutic agent for rheumatoid arthritis" is a biological drug product.

[Aspect 8] The therapeutic agent for rheumatoid arthritis, the use, the method, or the compound according to Aspect 5, wherein the "additional therapeutic agent for rheumatoid arthritis" is a JAK inhibitor.

**Effect of the Invention**

The present invention provides a therapeutic agent for rheumatoid arthritis that has been shown to be clinically effective in humans, is effective with short-term administration, and is highly safe.

**BRIEF DESCRIPTION OF THE DRAWINGS**

[Figure 1] Figure 1 shows the proportions of subjects who showed improvement in ACR20 in a working example. The horizontal axis indicates administration groups, and the vertical axis indicates the proportion for each administration group.

[Figure 2] Figure 2 shows the proportions of subjects who showed improvement in DAS28-CRP in a working example. The horizontal axis indicates administration groups, and the vertical axis indicates the proportion for each administration group.

[Figure 3] Figure 3 shows the VAS scale pain scores in a working example. The horizontal axis indicates administration groups, and the vertical axis indicates the percentage change in the VAS scale pain score from baseline (Day 1 of treatment) for each administration group.

[Figure 4] Figure 4 shows the TJC scores in a working example of the present invention. The horizontal axis indicates administration groups, and the vertical axis indicates the percentage change in the TJC from baseline (Day 1 of administration) for each administration group.

**EMBODIMENTS OF PRACTICING THE INVENTION**

Modes of practicing the present invention will here be described in detail.

The abbreviations used herein are defined as follows.

ACR20: An evaluation standard, set by the American College of Rheumatology, which corresponds to the ratio of cases in which, among the seven evaluation items included in the ACR core set (i.e., TJC, SJC, pain evaluation by patient (based on VAS; VAS scale pain score), PGA (based on VAS), EGA (based on VAS), physical function assessment by patient (based on HAQ), and CRP) both the TJC and the SJC have improved by at least 20% each, and at least three of the other five evaluation items have improved by at least 20%.

TJC: Tender joint count
SJC: Swollen joint count
VAS: Evaluation score based on visual analog scale
PGA: Patient's global assessment of disease activity
EGA: Evaluator's global assessment of disease activity
CRP: C-reactive protein (acute-phase reactant)
HAQ: Health assessment questionnaire
DAS28: Disease activity score (DAS) for 28 specific joints. When CRP (mg/dL) is used (DAS28-CRP), the calculation formula is defined as follows.

$$DAS28\text{-}CRP = 0.56 \times \sqrt{(TJC)} + 0.28 \times \sqrt{(SJC)} + 0.36 \times \ln((CRP) \times 10 + 1) + 0.14 \times (PGA)$$

(on a scale of 1 through 10) + 0.96 (note: "ln" represents the natural logarithm)

The present invention relates to a therapeutic agent for rheumatoid arthritis containing, as an active ingredient, (R)-1-(6-((6-(1-hydroxyethyl)-8-(isopropylamino)pyrido[3,4-d]pyrimidin-2-yl)amino)pyridin-3-yl)piperazin-2-one (Compound 1) or a pharmaceutically acceptable salt thereof.

According to the present invention, Compound 1 may also be in the form of a pharmaceutically acceptable salt. Examples include: inorganic acid salts such as hydrochlorides and phosphates; organic acid salts such as acetates and fumarates; salts with amino acids such as lysine and aspartic acid; salts with alkali metals such as sodium and potassium; salts with alkali rare-earth metals such as calcium and magnesium; salts with organic bases such as ethylamine and piperidine; and ammonium salts.

According to the present invention, pharmaceutically acceptable salts of Compound 1 include solvates with pharmaceutically acceptable solvents such as water and ethanol.

Compound 1 or a pharmaceutically acceptable salt thereof according to the present invention can be produced by any known method (e.g., the methods described in Patent Literature 1).

When used as a treatment for rheumatoid arthritis, the dosage of Compound 1 or a pharmaceutically acceptable salt thereof according to the present invention may be from 10 to 200 mg per day, preferably from 20 to 180 mg per day, more preferably from 30 to 125 mg per day, even more preferably from 60 to 90 mg per day, and particularly preferably 75 mg per day.

The therapeutic agent for rheumatoid arthritis according to the present invention may be used in various dosage forms, examples of which include tablets, pills, granules, pulverized agents, powders, fine granules, dry syrups, and capsules.

The therapeutic agent for rheumatoid arthritis according to the present invention may contain, in addition to Compound 1, additives normally used in the abovementioned dosage forms, such as excipients and binders. When the therapeutic agent is in the form of tablets, it may contain excipients such as lactose, binders such as hydroxypropyl methylcellulose, disintegrants such as sodium starch glycolate, and lubricants such as magnesium stearate.

The therapeutic agent for rheumatoid arthritis according to the present invention can be prepared by methods known to a person skilled in the art. When the therapeutic agent is in the form of tablets, they can be prepared by wet-granulating Compound 1, an excipient, and a binder with purified water, mixing the granules with a disintegrant and a lubricant, and tableting the mixture.

The therapeutic agent for rheumatoid arthritis according to the present invention is preferably administered orally.

The therapeutic agent for rheumatoid arthritis according to the present invention may be administered in combination with known therapeutic agents for rheumatoid arthritis, simultaneously, sequentially, or separately at predetermined intervals. Examples of known drugs for use in treating rheumatoid arthritis include: non-steroidal antiinflammatory drugs such as loxoprofen; steroids such as prednisolone; disease-modifying anti-rheumatic drugs (DMARDs) such as methotrexate and salazosulfapyridine; biological drug products such as infliximab, adalimumab, tocilizumab, etanercept, and abatacept; and JAK inhibitors such as tofacitinib, baricitinib, and filgotinib. Among various biological drug products, infliximab, adalimumab, etanercept, and the like are classified as TNF inhibitors; tocilizumab and the like are classified as IL-6 inhibitors; and abatacept and the like are classified as T-cell selective co-stimulatory modulators.

For example, the present invention may relate to a combination drug or kit for the treatment of rheumatoid arthritis containing, as active ingredients, (R)-1-(6-((6-(1-hydroxyethyl)-8-(isopropylamino)pyrido[3,4-d]pyrimidin-2-yl)amino)pyridin-3-yl)piperazin-2-one (Compound 1) or a pharmaceutically acceptable salt thereof and a known therapeutic agent for rheumatoid arthritis, such as methotrexate, etanercept, or tofacitinib. The present invention may also relate to use of (R)-1-(6-((6-(1-hydroxyethyl)-8-(isopropylamino)pyrido[3,4-d]pyrimidin-2-yl)amino)pyridin-3-yl)piperazin-2-one (Compound 1) or a pharmaceutically acceptable salt thereof in the manufacture of a combination drug or kit for the treatment of rheumatoid arthritis further containing a known therapeutic agent for rheumatoid arthritis. The known therapeutic agent for rheumatoid arthritis may be used at the concentrations normally used for rheumatoid arthritis treatment.

When used as a therapeutic agent for rheumatoid arthritis, Compound 1 may be administered once a day, but it can also be administered in two or three doses per day.

According to an embodiment, the present invention may include a method for treating rheumatoid arthritis by administering Compound 1 or a pharmaceutically acceptable salt thereof.

According to an embodiment, the present invention may include use of Compound 1 or a pharmaceutically acceptable salt thereof in the manufacture of a medicament for treating rheumatoid arthritis requiring treatment.

**WORKING EXAMPLES**

The present invention will now be described in further detail through working examples, but these working examples are presented merely for convenience of description and should not be construed as limitations to the present invention in any sense.

Working Example 1: Clinical trial on rheumatoid arthritis patients

1. Test method

Compound 1 was orally administered at dosages of 10, 25, 75, or 175 mg once daily for seven days (double-blind study) to 32 rheumatoid arthritis patients (six patients for each dosage of Compound 1 administration, and two patients for each dosage of placebo administration).

2. Endpoints

Endpoints included: items related to disease activity and pain (DAS28-CRP, ACR20, VAS scale pain score, and TJC) to represent efficacy; vital signs (blood pressure, pulse, body temperature, and respiratory rate), physical findings, electrocardiograms, hematological tests, blood biochemistry tests, and the severity and frequency of adverse events to represent safety; and changes in the plasma concentration of Compound 1 to represent pharmacokinetics (PK).

DAS28-CRP was used to determine whether there was improvement or no improvement based on the differences between the values on Day 1 and Day 7 of administration, in accordance with the criteria shown in Table 1 (EULAR criteria).

[Table 1]

| DAS28-CRP (Day 7 of administration) | Difference between Day 1 and Day 7 of administration | | |
|---|---|---|---|
| | > 1.2 | 0.6 to 1.2 | ≤ 0.6 |
| ≤ 3.2 | Significant improvement | Moderate improvement | No response (no improvement) |
| 3.2 to 5.1 | Moderate improvement | Moderate improvement | No response (no improvement) |
| > 5.1 | Moderate improvement | No response (no improvement) | No response (no improvement) |

ACR20 was used to determine whether there was an improvement based on the ACR20 on Day 1 and Day 7 of administration.

The percentage change in the VAS scale pain score is the percentage change in the score on Day 7 compared to the score on Day 1.

The percentage change in the TJC score is the percentage change in the value on Day 7 compared to the value on Day 1.

3. Analysis results

(1) Efficacy

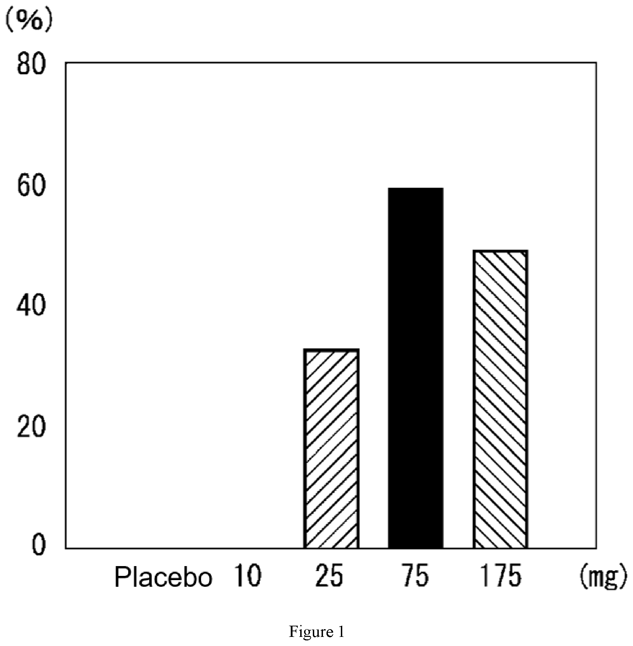

Figure 1 shows the percentage of subjects in each administration group who showed improvement in ACR20 after seven days of oral administration.

The percentages of subjects who showed improvement in the 25 mg, 75 mg, and 175 mg administration groups were higher than in the placebo group, indicating a superior improvement effect on rheumatoid arthritis. In particular, the 75 mg administration group showed a particularly significant effect, with 60% of the subjects showing improvement.

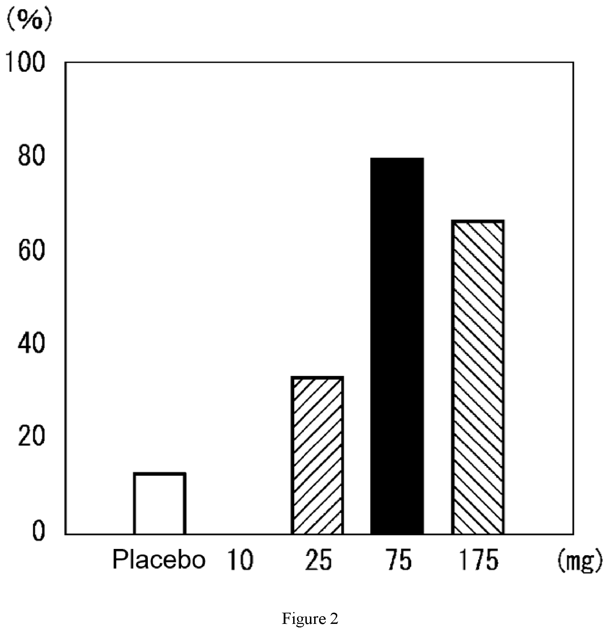

Figure 2 shows the percentage of subjects in each administration group who showed improvement in DAS28-CRP after seven days of oral administration.

The percentages of subjects who showed improvement in the 25 mg, 75 mg, and 175 mg administration groups were higher than in the placebo group, indicating a superior improvement effect on rheumatoid arthritis. In particular, the 75 mg administration group showed a particularly significant effect, with 80% of the subjects showing improvement.

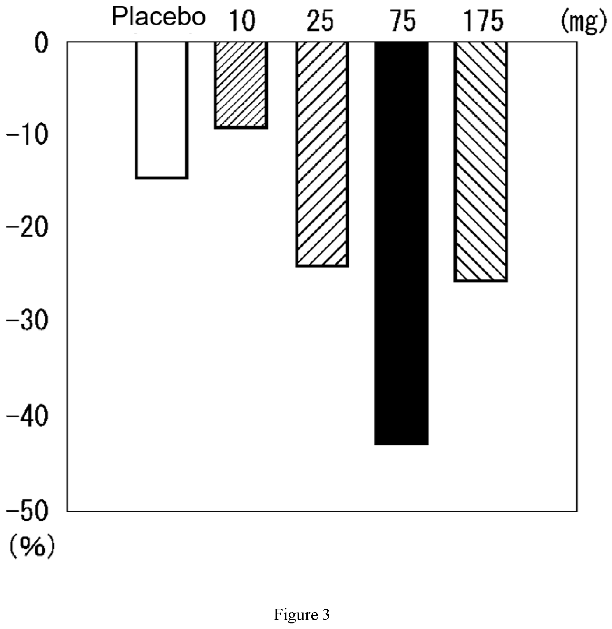

Figure 3 shows the percentage change in the VAS scale pain score in each administration group.

The decreases in the scores of the subjects in the 25 mg, 75 mg, and 175 mg administration groups were higher than in the placebo group, indicating a superior improvement effect on rheumatoid arthritis. In particular, the 75 mg administration group showed a particularly significant effect, with the percentage change being -40%.

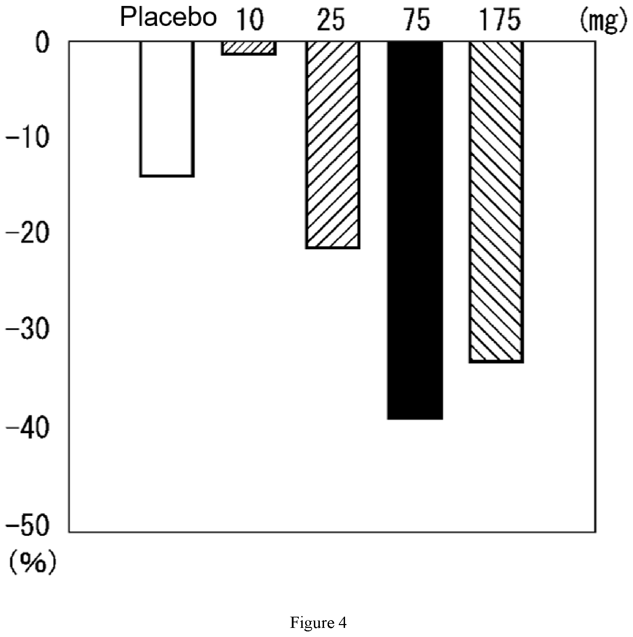

Figure 4 shows the percentage change in the TJC score in each administration group.

The decreases in the scores of the subjects in the 25 mg, 75 mg, and 175 mg administration groups were higher than in the placebo group, indicating a superior improvement effect on rheumatoid arthritis. In particular, the 75 mg administration group showed a particularly significant effect, with the percentage change being -40%.

(2) Safety

No significant changes were observed in vital signs, electrocardiograms, physical findings, hematological tests, or blood biochemistry tests at any dosage. With regard to adverse events, two cases (five events) were reported in the 10 mg administration group, two cases (three events) were reported in the 25 mg administration group, two cases (three events) were reported in the 75 mg administration group, and one case (one event) was reported in the 175 mg administration group, but all were mild to moderate, and there were no severe adverse events.

Existing CDK4/6 inhibitors (palbociclib and abemaciclib) are associated with a high incidence of the side effect of neutropenia (palbociclib: 80.2% (package insert), abemaciclib: 42.2% (package insert)). On the other hand, Compound 1 maintained a constant neutrophil count from Day 1 to Day 7 of administration and throughout the three-day follow-up period after administration (i.e., up to Day 10 from Day 1 of administration), and there was no neutropenia or decrease in neutrophil count.

(3) Pharmacokinetics

The half-life of Compound 1 in plasma on Day 7 after administration in each administration group was 12.9 hours for 10 mg, 11.9 hours for 25 mg, 14.6 hours for 75 mg, and 10.4 hours for 175 mg, with the longest half-life observed in the 75 mg formulation.

Working Example 2: Pharmacological testing using synovial cells (*in vitro*)

The synovial cells used in the test were derived from rheumatoid arthritis patients. The synovial cells were stimulated with tumor necrosis factor-$\alpha$ (TNF-$\alpha$) and interleukin-1$\beta$ (IL-1$\beta$) at the time of the test, and the effect of Compound 1 was measured. The results showed that, relative to a baseline after TNF-$\alpha$ and IL-1$\beta$ stimulation without any added Compound 1, the proliferation of synovial cells was suppressed in a dosage-dependent manner, by 5% when 10 nM was added, 14% when 30 nM was added, 19% when 100 nM was added, 27% when 300 nM was added, and 35% when 1000 nM was added. In addition, with regard to granulocyte macrophage colony-stimulating factor (GM-CSF), which is an inflammatory mediator, relative to a baseline after TNF-$\alpha$ and IL-1$\beta$ stimulation without any added Compound 1, Compound 1 suppressed secretion in a dosage-dependent manner, by 17% when 8 nM was added, 25% when 40 nM was added, 48% inhibition when 200 nM was added, and 65% when 1000 nM was added. Furthermore, with regard to matrix metalloproteinase-3 (MMP-3), which is a mediator involved in tissue destruction, relative to a baseline after TNF-$\alpha$ and IL-1$\beta$ stimulation without any added Compound 1, Compound 1 suppressed secretion in a dosage-dependent manner, by 14% when 10 nM was added, 21% when 30 nM was added, 37% when 100 nM was added, 60% when 300 nM was added, and 63% when 1000 nM was added.

Working Example 3: Distribution test in synovial cells (*in vitro*)

Cultured synovial cells and bone marrow cells were used. Compound 1 was added, and the intracellular concentration in the synovial cells and bone marrow cells and the concentration in the culture medium were measured. The results showed that Compound 1 was distributed selectively to synovial cells, and the distribution to bone marrow cells was extremely low compared to the distribution to synovial cells (the distribution ratio between synovial cells and bone marrow cells was 84:16). Palbociclib, a CDK4/6 inhibitor with the same mechanism of action, did not show any specific distribution (the distribution ratio between synovial cells and bone marrow cells was 43:57), indicating that Compound 1 showed different distribution characteristics.

Working Example 4: Combination study with methotrexate (*in vivo*)

The effect of combining Compound 1 with methotrexate was measured using a rat adjuvant-induced arthritis (AIA) model. The effect was evaluated using the Arthritis Score. The results showed that when Compound 1 (30 mg/kg/day) and methotrexate (0.075 mg/kg/day) were used in combination, the effect was much higher than that shown by Compound 1 alone or by methotrexate alone. Compared to the control, the scores decreased by 33% when only Compound 1 was used, by 59% when only methotrexate was used, and by 86% when Compound 1 and methotrexate were used in combination. On the other hand, in this study, there was no decrease in the number of bone marrow cells even when Compound 1 and methotrexate were used in combination.

Working Example 5: Combination study with etanercept (*in vivo*)

The effect of combining Compound 1 with etanercept was measured using a mouse anti-collagen antibody-induced arthritis (CAIA) model. The effect was evaluated using the Arthritis Score. The results showed that when Compound 1 (50 mg/kg/day) and etanercept (10 mg/kg/day) were used in combination, the effect was much higher than that shown by Compound 1 alone or etanercept alone. Compared to the control, the scores decreased by 64% when only Compound 1 was used, by 62% when only etanercept was used, and by 97% when Compound 1 and etanercept were used in combination.

Working Example 6: Combination study with tofacitinib (*in vivo*)

The effect of combining compound 1 with tofacitinib was measured using a rat AIA model. The effect was evaluated using the Arthritis Score. The results showed that when Compound 1 (30 mg/kg/day) and tofacitinib (30 mg/kg/day) were used in combination, the effect was much higher than that shown by Compound 1 alone or tofacitinib alone. Compared to the control, the score decreased by 40% when only Compound 1 was used, by 67% when only tofacitinib was used, and by 92% when Compound 1 and tofacitinib were used in combination.

Based on the results from the working examples, Compound 1 was effective in treating rheumatoid arthritis in humans, and the effect was particularly high in the 75 mg administration group. There were no safety issues with any of the dosages.

**INDUSTRIAL APPLICABILITY**

The present invention is extremely useful as a therapeutic agent for rheumatoid arthritis.

**Claims**

1. A therapeutic agent for rheumatoid arthritis comprising (R)-1-(6-((6-(1-hydroxyethyl)-8-(isopropylamino)pyrido[3,4-d]pyrimidin-2-yl)amino)pyridin-3-yl)piperazin-2-one or a pharmaceutically acceptable salt thereof.

2. The therapeutic agent for rheumatoid arthritis according to claim 1, wherein the daily dosage of the compound is from 10 to 200 mg.

3. The therapeutic agent for rheumatoid arthritis according to claim 1, wherein the daily dosage of the compound is from 30 to 125 mg.

4. The therapeutic agent for rheumatoid arthritis according to any one of claims 1 through 3, wherein the therapeutic agent for rheumatoid arthritis is administered once a day.

5. The therapeutic agent for rheumatoid arthritis according to any one of claims 1 through 4, wherein the therapeutic agent for rheumatoid arthritis is used in combination with an additional therapeutic agent for rheumatoid arthritis.

6. The therapeutic agent for rheumatoid arthritis according to claim 5, wherein the "additional therapeutic agent for rheumatoid arthritis" is a disease-modifying anti-rheumatic drug (DMARD).

7. The therapeutic agent for rheumatoid arthritis according to claim 5, wherein the "additional therapeutic agent for rheumatoid arthritis" is a biological drug product.

8. The therapeutic agent for rheumatoid arthritis according to claim 5, wherein the "additional therapeutic agent for rheumatoid arthritis" is a JAK inhibitor.

Figure 1

Figure 2

Figure 3

Figure 4

## INTERNATIONAL SEARCH REPORT

| International application No. |
|---|
| **PCT/JP2024/034410** |

| A. | CLASSIFICATION OF SUBJECT MATTER |
|---|---|

*A61K 31/519*(2006.01)i; *A61P 19/02*(2006.01)i; *A61P 29/00*(2006.01)i
FI:   A61K31/519; A61P29/00 101; A61P19/02

According to International Patent Classification (IPC) or to both national classification and IPC

| B. | FIELDS SEARCHED |
|---|---|

Minimum documentation searched (classification system followed by classification symbols)

A61K31/519; A61P19/02; A61P29/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

| C. | DOCUMENTS CONSIDERED TO BE RELEVANT | |
|---|---|---|

| Category* | Citation of document, with indication, where appropriate, of the relevant passages | Relevant to claim No. |
|---|---|---|
| A | WO 2016/194831 A1 (TEIJIN PHARMA LIMITED) 08 December 2016 (2016-12-08) claims, examples | 1-8 |
| A | WO 2018/097295 A1 (TEIJIN PHARMA LIMITED) 31 May 2018 (2018-05-31) claims, examples | 1-8 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 December 2024** | **17 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/034410**

| Patent document cited in search report | | | Publication date (day/month/year) | Patent family member(s) | | | Publication date (day/month/year) |
|---|---|---|---|---|---|---|---|
| WO | 2016/194831 | A1 | 08 December 2016 | US | 2018/0161329 | A1 | |
| | | | | claims, examples | | | |
| | | | | EP | 3305785 | A1 | |
| | | | | CN | 107614499 | A | |
| | | | | KR | 10-2018-0011122 | A | |
| WO | 2018/097295 | A1 | 31 May 2018 | US | 2021/0276997 | A1 | |
| | | | | claims, examples | | | |
| | | | | EP | 3546456 | A1 | |
| | | | | KR | 10-2019-0067247 | A | |
| | | | | CN | 109996800 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016194831 A

**Non-patent literature cited in the description**

- *Ann Rheum Dis*, 2017, vol. 76, 1009-1019